Europäisches Patentamt

(19) European Patent Office

Office européen des brevets

(11) EP 0 667 144 B1

(12) **FASCICULE DE BREVET EUROPEEN**

(45) Date de publication et mention de la délivrance du brevet:
**12.08.1998 Bulletin 1998/33**

(51) Int. Cl.$^6$: **A61K 7/42**

(21) Numéro de dépôt: **95400095.6**

(22) Date de dépôt: **18.01.1995**

(54) **Compositions cosmétiques antisolaires**

Kosmetische Sonnenschutzmittel

Sunscreening cosmetic compositions

(84) Etats contractants désignés:
**DE ES FR GB IT**

(30) Priorité: **09.02.1994 FR 9401455**

(43) Date de publication de la demande:
**16.08.1995 Bulletin 1995/33**

(73) Titulaire: **L'OREAL**
**75008 Paris (FR)**

(72) Inventeurs:
• **Allard, Delphine**
**F-92700 Colombes (FR)**
• **Ascione, Jean-Marc**
**F-75018 Paris (FR)**
• **Hansenne, Isabelle**
**F-75017 Paris (FR)**

(74) Mandataire:
**Andral, Christophe André Louis**
**L'OREAL**
**Centre de Recherche Charles Zviak**
**Département Propriété Industrielle**
**90, rue du Général Roguet**
**92583 Clichy Cedex (FR)**

(56) Documents cités:
**EP-A- 0 518 773**      **EP-A- 0 559 319**
**WO-A-92/10995**

## Description

La présente invention concerne de nouvelles compositions cosmétiques à usage topique plus particulièrement destinées à la photoprotection de la peau et/ou des cheveux contre le rayonnement ultraviolet (compositions ci-après dénommées plus simplement compositions antisolaires), leur procédé de préparation, ainsi que leur utilisation dans l'application cosmétique susmentionnée. Plus précisément encore, elle concerne des compositions antisolaires à propriétés améliorées se présentant sous la forme d'émulsions de type huile-dans-eau (support cosmétiquement acceptable) et comprenant, à titre d'agents photoprotecteurs agissant par blocage physique du rayonnement (réflecteurs et/ou diffuseurs d'UV), des nanopigments minéraux à base d'oxydes métalliques, et en particulier d'oxyde de titane.

Il est bien connu que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel; ce rayonnement UV-B doit donc être filtré.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.

De nombreuses compositions destinées à la photoprotection (UV-A et/ou UV-B) de la peau ont été proposées à ce jour et, dans ce domaine, on observe que l'utilisation de nanopigments minéraux (c'est à dire de pigments dont la taille moyenne des particules primaires n'excède généralement pas 100 nm) à base d'oxydes métalliques, et en particulier d'oxyde de titane, devient de plus en plus fréquente, compte tenu du fait que ces dernières substances, lorsqu'elles sont associées avec des filtres UV classiques (principalement des composés organiques capables d'absorber les rayonnements nocifs) permettent d'obtenir des indices de protection très élevés.

Pour diverses raisons liées en particulier à un meilleur confort d'utilisation (douceur, émollience et autres), les compositions antisolaires actuelles se présentent le plus souvent sous la forme d'une émulsion de type huile-dans-eau (c'est à dire un support constitué d'une phase continue dispersante aqueuse et d'une phase discontinue dispersée huileuse) dans laquelle on a introduit, à des concentrations diverses, les nanopigments susmentionnés, le cas échéant en association avec d'autres filtres UV classiques, et qui peuvent être présents tant dans la phase aqueuse de l'émulsion que dans sa phase huileuse (encore appelée phase grasse). Dans ces émulsions classiques, qui contiennent en outre des agents émulsionnants (ou tensio-actifs) et d'éventuels additifs cosmétiques usuels tels que parfums, colorants ou conservateurs, la taille des globules constituant la phase grasse est généralement supérieure à plusieurs microns.

L'un des inconvénients majeurs des compositions antisolaires connues à ce jour et appartenant au type ci-dessus (émulsion H/E contenant des nanopigments), et plus particulièrement de celles contenant des nanopigments d'oxyde de titane TiO$_2$, est que, une fois appliquées sur la peau sous la forme d'un film, elles engendrent sur cette dernière un effet de blanchissement qui est cosmétiquement indésirable et généralement peu apprécié des utilisateurs. Cet effet est d'autant plus marqué que la concentration en nanopigments dans l'émulsion est élevée. Pour éviter ce problème, il serait bien entendu possible de mettre en oeuvre des quantités réduites de nanopigments, mais les émulsions résultantes, qui conduiraient certes à des films présentant une transparence acceptable sur la peau, n'offriraient alors plus une protection convenable dans le domaine des UV, ce qui limite fortement l'intérêt d'une telle opération.

Une autre difficulté réside par ailleurs dans le fait que les émulsions classiques antisolaires à base de nanopigments protecteurs conduisent, après application sur la peau. à une distribution irrégulière, non homogène, voire grossière, desdits nanopigments sur cette peau, ce qui peut nuire à la qualité de l'effet de photoprotection global recherché. Cette mauvaise répartition des nanopigments que l'on constate à la surface de la peau est souvent liée au fait qu'il existe, au niveau de l'émulsion initiale même (avant application), un manque substantiel d'homogénéité (mauvaise dispersion du pigment dans son support).

Enfin, on observe, pour certaines des émulsions antisolaires ci-dessus, et bien que ces dernières contiennent des agents émulsionnants (ou tensio-actifs) comme indiqué précédemment, un certain manque de stabilité dans le temps, ce qui nuit à leur conservation une fois conditionnées. Ce manque de stabilité se traduit, dans les faits, par des phénomènes plus ou moins prononcés de décantation des nanopigments au sein de l'émulsion, voire de séparation entre les phases aqueuse et huileuse de cette émulsion.

La présente invention a notamment pour but de résoudre les problèmes ci-dessus.

Plus précisémment encore, la présente invention vise à proposer des émulsions à propriétés antisolaires de type H/E et contenant des nanopigments minéraux à base d'oxydes métalliques, qui présentent à la fois une excellente transparence sur la peau, une très bonne efficacité de protection contre les UV, une très bonne stabilité et une parfaite homogénéité tant avant qu'après application sur la peau (c'est à dire que les nanopigments sont très bien dispersés dans l'émulsion initiale d'une part et sur la peau après application d'autre part).

A la suite d'importantes recherches menées sur la question, il a maintenant été trouvé par la Demanderesse, et ceci de façon tout à fait inattendue et surprenante, qu'il est possible de remédier aux différents inconvénients liés à l'emploi et à la présence de nanopigments photoprotecteurs dans les émulsions H/E classiques de l'art antérieur, en mettant en oeuvre des émulsions H/E spécifiques dites "ultrafines", pour lesquelles la taille des globules constituant la phase grasse est comprise dans des limites bien déterminées, lesdites émulsions ultrafines de type H/E étant elles-mêmes de préférence obtenues selon la technique dite "d'inversion de phase" qui sera détaillée par la suite. Toutes choses étant égales par ailleurs (i.e. à composition chimique et concentrations identiques), on observe qu'une composition antisolaire conforme à l'invention, par le simple ajustement de la taille des globules huileux à une valeur convenable indiquée ci-après, présente systématiquement, au niveau notamment de sa tranparence sur la peau, de sa stabilité, de son homogéïté et de son pouvoir de protection, des propriétés améliorées par rapport à une même composition antisolaire ne satisfaisant pas au critère susmentionné de taille des globules d'huile.

Cette découverte est à la base de la présente invention.

Ainsi, conformément à l'un des objets de la présente invention, il est maintenant proposé de nouvelles compositions cosmétiques, en particulier antisolaires, stables et homogènes, comprenant, dans un support cosmétiquement acceptable de type émulsion huile-dans-eau, des nanopigments minéraux à base d'oxydes métalliques à titre d'agents photoprotecteurs, et qui sont caractérisées par le fait que la taille moyenne des globules qui constituent la phase huileuse de ladite émulsion est comprise entre 100 nm et 1000 nm.

D'autres caractéristiques, aspects et avantages de l'invention apparaitront à la lecture de la description détaillée qui va suivre.

Les oxydes métalliques utilisables dans le cadre de la présente invention sont tous ceux déja connus en soi pour leur activité photoprotectrice. Ainsi, ils peuvent être notamment choisis parmi les oxydes de titane, de zinc, de fer, de zirconium, de cérium ou leurs mélanges.

De tels nanopigments d'oxydes métalliques, enrobés ou non enrobés, sont des produits déja bien connus de l'homme de l'art et sont en particulier décrits dans la demande de brevet EP-A- 0 518 773. A titre de nanopigments commerciaux complémentaires non cités dans la demande précitée mais également utilisables dans le cadre de la présente invention, on peut en outre mentionner les produits vendus sous les noms de marque UVT M 160, UVT M 212 et UVT M 262 par la Société KEMIRA, et MT 100 SAS par la Société TAYCA.

Selon un mode préféré de réalisation des compositions antisolaires selon l'invention, on fait appel à des nanopigments minéraux à base d'oxyde de titane, qui offrent en effet la meilleure efficacité au niveau photoprotection. Par ailleurs, on notera que l'effet cosmétique indésirable de blanchissement de la peau évoqué dans la partie introductive de la présente description est particulièrement marqué avec ce type de nanopigments. Cet oxyde de titane peut se présenter sous une forme cristallisée de type rutile et/ou anatase, et/ou sous une forme amorphe ou substantiellemnt amorphe. Comme indiqué précédemment, ce pigment peut être alors enrobé ou non enrobé, mais de préférence on utilise des pigments enrobés, par exemple par de l'alumine et/ou du stéarate d'aluminium.

Selon leur caractère lipophile, ou au contraire hydrophile, plus ou moins prononcé, les nanopigments pourront être présents soit dans la phase grasse de l'émulsion, soit dans la phase aqueuse, ou bien même encore dans les deux phases à la fois.

La taille moyenne des particules primaires des nanopigments présents dans les compositions selon l'invention est généralement comprise entre 5 nm et 100 nm, de préférence entre 10 et 50 nm.

Bien entendu, les compositions antisolaires conformes à l'invention peuvent en outre contenir un ou plusieurs filtres solaires organiques classiques (absorbeurs), actifs dans l'UV-A et/ou l'UV-B, hydrophiles ou lipophiles. A titre d'exemples, ces filtres complémentaires peuvent être choisis parmi l'acide 2-phényl benzimidazole 5-sulfonique et ses sels, les dérivés cinnamiques tels que par exemple le p-méthoxycinnamate de 2-éthylhexyle, les dérivés salicyliques comme par exemple le salicylate de 2-éthylhexyle et le salicylate d'homomenthyle, les dérivés du camphre comme par exemple le 3(4-méthylbenzylidène)camphre ou l'acide camphosulfonique (1,4 divinylbenzène), les dérivés de triazine tels que la 2,4,6-tris [p-(2'-éthylhexyl-1'-oxycarbonyl)anilino] 1,3,5-triazine, les dérivés de la benzophénone tels que la 2-hydroxy 4-méthoxybenzophénone, les dérivés du dibenzoylméthane tels que le 4-tert-butyl 4'-méthoxyd ibenzoylméthane, les dérivés de β,β-diphénylacrylate tels que le α-cyano-β,β-diphénylacrylate de 2-éthylhexyle, les dérivés de l'acide p-aminobenzoïque comme par exemple le paradiméthylaminobenzoate d'octyle, l'anthranilate de menthyle, les polymères filtres et silicones filtres décrits dans la demande WO-93-04665. D'autres exemples de filtres organiques sont donnés dans la demande de brevet EP-A 0 487 404.

La nature de la phase grasse rentrant dans la composition des émulsions selon l'invention n'est pas critique et elle peut ainsi être constituée par tous les composés qui sont déjà connus de façon générale comme convenant pour la fabrication d'émulsions de type huile dans eau. En particulier, ces composés peuvent être choisis, seuls ou en mélanges, parmi les différents corps gras, les huiles d'origine végétale, animale ou minérale, les cires naturelles ou synthétiques, et analogues.

Parmi les huiles pouvant rentrer dans la composition de la phase grasse, on peut notamment citer :

- les huiles minérales telles que l'huile de paraffine et l'huile de vaseline,
- les huiles d'origine animale telles que le perhydrosqualéne,
- les huiles d'origine végétale telles que l'huile d'amande douce, l'huile d'avocat, l'huile de ricin, l'huile d'olive, l'huile de jojoba, l'huile de sésame, l'huile d'arachide, l'huile de pépins de raisin, l'huile de colza, l'huile de coprah, l'huile de noisette, le beurre de karité, l'huile de palme, l'huile de noyau d'abricot, l'huile de calophyllum, l'huile de son de riz, l'huile de germes de maïs, l'huile de germes de blé, l'huile de soja, l'huile de tournesol, l'huile d'onagre, l'huile de carthame, l'huile de passiflore et l'huile de seigle,
- les huiles synthétiques telles que l'huile de purcellin, le myristate de butyle, le myristate d'isopropyle, le myristate de cétyle, le palmitate d'isopropyle, l'adipate d'isopropyle, l'adipate d'éthylhéxyle, le stéarate de butyle, le stéarate d'héxadécyle, le stéarate d'isopropyle, le stéarate d'octyle, le stéarate d'isocétyle, l'oléate de décyle, le laurate d'héxyle, le dicaprylate de propylène glycol et les esters dérivés d'acide lanolique tels que le lanolate d'isopropyle, le lanolate d'isocétyle, les isoparaffines et les poly-$\alpha$-oléfines.

Comme autres huiles utilisables dans les émulsions selon l'invention, on peut encore citer les benzoates d'alcools gras en $C_{12}$-$C_{15}$ (Finsolv TN de FINETEX), les alcools gras tels que l'alcool laurique, cétylique, myristique, stéarique, palmitique, oléique ainsi que le 2-octyldodécanol, les acétylglycérides, les octanoates et décanoates d'alcools et de polyalcools tels que ceux de glycol et de glycérol, les ricinoléates d'alcools et de polyalcools tels que ceux de cétyle, les triglycérides d'acides gras tels que les triglycérides caprylique/caprique, les triglycérides d'acides gras saturés en$C_{10}$-$C_{18}$, les huiles fluorées et perfluorées, la lanoline, la lanoline hydrogénée, la lanoline acétylée et enfin les huiles de silicones, volatiles ou non.

Bien entendu, la phase grasse peut également contenir un ou plusieurs adjuvants cosmétiques lipophiles classiques, notamment ceux qui sont déja utilisés de manière habituelle dans la fabrication et l'obtention des compositions cosmétiques antisolaires.

Selon une caractéristique essentielle de la présente invention, la taille moyenne des particules liquides (ou globules) de phase grasse au sein de la phase aqueuse dispersante doit être comprise dans des limites bien particulières, à savoir entre 100 nm et 1000 nm. De préférence, cette taille moyenne est comprise entre 100 nm et 500 nm. Encore plus préférentiellement, la distribution en taille des globules huileux est telle que la plupart desdits globules (i.e au moins 90% en nombre) présentent une taille comprise entre les bornes indiquées ci-avant.

De manière classique, la phase aqueuse dispersante peut être constituée par de l'eau, ou un mélange d'eau et d'alcool(s) polyhydrique(s) comme par exemple glycérol, propyléneglycol et sorbitol, ou bien encore un mélange d'eau et d'alcool(s) inférieur(s) hydrosoluble(s) tels que éthanol, isopropanol ou butanol (solution hydroalcoolique), et elle peut bien entendu en outre contenir des adjuvents cosmétiques classiques hydrosolubles.

Parmi les adjuvants cosmétiques classiques susceptibles d'être contenus dans la phase aqueuse et/ou dans la phase grasse des émulsions conformes à l'invention (selon leur caractère hydro- et/ou liposoluble), on peut citer notamment les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants, les colorants, ou tout autre ingrédient habituellement utilisé dans le domaine des produits solaires.

Les émulsions conformes à l'invention contiennent en outre généralement des tensio-actifs ou émulsionnants particuliers dont l'emploi a été rendu nécessaire pour la préparation et l'obtention de l'émulsion ultrafine. Ce point sera détaillé par la suite. Elles peuvent en outre contenir des co-émulsionnants spécifiques dont le rôle est, lors de la préparation de l'émulsion, de diminuer de manière substantielle la quantité d'agents tensio-actifs nécessaire à la réalisation de l'émulsion.

A titre indicatif, les formulations antisolaires conformes à l'invention présentent généralement les compositions suivantes :

(i) phase aqueuse : de 50 à 95 % en poids, de préférence de 70 à 90 % en poids, par rapport à l'ensemble de la formulation,

(ii) phase huileuse : de 5 à 50 % en poids, de préférence de 10 à 30 % en poids, par rapport à l'ensemble de la formulation,

(iii) nanopigments : de 0,5 à 40 % en poids, de préférence de 1 à 30 % en poids, par rapport à l'ensemble de la formulation,

(iv) (co)émulsionnant(s) : de 0,5 à 20 % en poids, de préférence de 2 à 10% en poids, par rapport à l'ensemble de la formulation.

Le procédé préféré de préparation des compositions selon l'invention, lui-même constitutif d'un second objet de la présente invention, va maintenant être développé.

Comme indiqué précédemment, ce procédé repose sur la technique de fabrication des émulsions H/E par inversion de phase. Cette technique est, dans son principe, bien connue de l'homme de l'art et est notamment décrite dans l'article "Phase Inversion Emusification", par Th Förster et al, paru dans Cosmetics & Toiletries, vol. 106, Decembre 1991, pp 49-52. Son principe est ainsi le suivant : on prépare une émulsion (introduction de l'eau dans l'huile) à une température qui doit être supérieure à la température d'inversion de phase (ou TIP) du système, c'est à dire la température à laquelle l'équilibre entre les propriétés hydrophiles et lipophiles du ou des émulsionnants mis en oeuvre est atteint à température élevée (>TIP), l'émulsion est de type eau-dans-huile, et au cours de son refroidissement, à la température d'inversion de phase, cette émulsion s'inverse pour devenir une émulsion de type cette fois huile-dans-eau, et ceci en étant passée auparavant par un état de microémulsion.

Selon une caractéristique essentielle de l'invention, des nanopigments doivent être présents dans l'émulsion ultrafine H/E finale. Ainsi, selon une première variante de mise en oeuvre du procédé de préparation selon l'invention, l'inversion de phase de l'émulsion est conduite en présence des nanopigments photoprotecteurs décrits précédemment ; selon une deuxième variante de mise en oeuvre de ce procédé, ces nanopigments ne sont introduits qu'après que l'émulsion par inversion de phase ait été obtenue. Il est bien entendu possible de cumuler les deux variantes.

L'une des difficultés pour la mise en oeuvre d'un procédé tel que ci-dessus réside dans le choix convenable du système émulsionnant qui doit être approprié au résultat recherché.

Les systèmes émulsionnants qui doivent ainsi être retenus dans le cadre de l'invention sont ceux qui permettent effectivement d'obtenir des émulsions ultrafines par inversion de phase (100 nm<$\Phi_{globules}$<1000 nm), stables, et dans lesquelles les nanopigments se trouvent dispersés de façon fine et homogène.

Les travaux de la Demanderesse ont montré que, à cet effet, les systèmes émulsionnants convenant à la présente invention devraient être des émulsionnants de type non ioniques et, plus particulièrement encore, être choisis parmi les alcools gras polyoxyéthylénés et/ou polyoxypropylénés (i.e des composés obtenus par réaction entre un alcool gras aliphatique, comme l'alcool béhénique ou l'alcool cétylique, avec de l'oxyde d'éthylène ou de l'oxyde de propylène ou un mélange oxyde d'éthylène/oxyde de propylène) et les esters d'acides gras et de polyols, éventuellement polyoxyéthylénés et/ou polyoxypropylénés (i.e des composés obtenus par réaction d'un acide gras, comme l'acide stéarique ou l'acide oléique, avec un polyol, comme par exemple un alkyléneglycol ou du glycérol ou un polyglycérol, éventuellement en présence d'oxyde d'éthylène ou d'oxyde de propylène ou d'un mélange oxyde d'éthylène/oxyde de propylène), ou leurs mélanges. Par ailleurs, et de préférence, le système émulsionnant retenu présentera un HLB global (comme cela est bien connu, on désigne par HLB (Hydrophilic-Lipophilic Balance, au sens de Griffin ; voir J. Soc. Cosm. Chem. 1954 (vol 5), pp 249-256) l'équilibre entre le caractère hydrophile et le caractère lipophile de l'agent tensioactif) compris entre 9,5 et 11,5 environ, avantageusement proche de 10, de manière à permettre l'obtention d'une inversion de phase à une température inférieure à 90°C (TIP<90°C).

De façon inattendue et surprenante, la présence des nanopigments minéraux dans le système initial à émulsionner ne perturbe en rien les mécanismes qui sont naturellement mis en jeu dans un procédé d'émulsification par inversion de phase; au contraire, on aboutit à une émulsion ultrafine dans laquelle les particules constituant les nanopigments sont elles-mêmes maintenues à l'état d'une fine dispersion (absence d'agglomération, ou taille d'agglomérats extrêmement faible), parfaitement homogène et stable dans le temps.

Les détails du procédé de préparation selon l'invention apparaitront dans les exemples donnés ci-après.

Un troisième objet de la présente invention est constitué par l'utilisation des compositions selon l'invention telles que ci-dessus définies pour la fabrication de formulations photoprotectrices de l'épiderme humain ou des cheveux contre les rayons ultraviolets, en particulier le rayonnement solaire. Les compositions peuvent alors être conditionnées sous la forme de crèmes, de lait, de gels crèmes, ou bien encore de lotions fluides, en particulier de lotions fluides vaporisables (les compositions selon l'invention présentant en effet la propriété avantageuse complémentaire d'être aisément diluables à l'eau).

Le procédé de traitement cosmétique de la peau ou des cheveux destiné à les protéger contre les effets des rayons UV, en particulier de ceux contenus dans le rayonnement solaire, consiste à appliquer sur ceux-ci une quantité efficace d'une composition cosmétique telle que définie ci-dessus.

Des exemples concrets, mais nullement limitatifs, illustrant l'invention, vont maintenant être donnés.

## EXEMPLE 1

Dans cet exemple, on a préparé et comparé deux formulations de même composition chimique et contenant des nanopigments de $TiO_2$, l'une étant conforme à l'invention (F1), ultrafine et obtenue par inversion de phase, l'autre comparative (F2), non ultrafine ($\Phi_{globules}$>1 $\mu$m) et obtenue sans mettre en oeuvre d'inversion de phase.

Le mode opératoire qui a été suivi pour préparer ces deux compositions était le suivant : les phases grasse (A) et aqueuse (C) ont été toutes deux préalablement portées à une température de l'ordre de 90°C ; puis on a introduit et dispersé la phase (B) contenant les nanopigments dans la phase grasse (A), et ceci sous agitation énergique de cette dernière au moyen d'une turbine type MORITZ (1000 t/mn) ; et enfin on a ajouté la phase aqueuse (A) dans la dispersion résultante, toujours sous agitation mécanique : dans le cas de la formulation (1), cette dernière étape d'émulsification a été conduite à 80°C, c'est à dire à une température supérieure à la température d'inversion de phase du système, laquelle est ici de 72°C (TIP), alors que dans le cas de la formulation (2), on a conduit l'émulsification à 55°C (i.e. T°C<TIP).

Les compositions chimiques (% en poids ramenés à l'ensemble de la formulation) étaient les suivantes :

| Phase A : | |
|---|---|
| - Alcool cétylstéarylique à 15 OE, vendu sous le nom de MERGITAL® CS 15 par HENKEL | 6,6 % |
| - Stéarate de glycérol, vendu sous le nom de TEGIN® 90 par GOLDSCHMIDT | 3,4 % |
| - Huile de vaseline | 45,0 % |

| Phase B : | |
|---|---|
| - oxyde de titane TiO2 nanopigmentaire, vendu sous la référence MT 100T par la Société TAYCA | 5 % |

| Phase C : | | |
|---|---|---|
| - Glycérine | | 5 % |
| - Conservateurs | | qs |
| - Eau | qsp | 100 % |

Pour les formulations 1 et 2 ainsi obtenues, on a ensuite comparé :

- les caractéristiques des émulsions au niveau de la taille des globules d'huile (microscope optique G x 400)
- la qualité de dispersion du pigment dans l'émulsion (microscope optique G x 100)
- leur stabilité à la chaleur d'une part (45°C) et à la centrifugation d'autre part (30 min à 3000 trs/min).

Les résultats sont rassemblés dans le tableau I ci-dessous. Ceux-ci démontrent clairement la supériorité de la formulation conforme à l'invention au niveau de la qualité de la dispersion du pigment d'une part et de la stabilité d'autre part.

On a par ailleurs évalué les deux formulations F1 et F2 au niveau de leur transparence sur la peau de trois personnes témoins (P1, P2 et P3). On a ainsi appliqué ces formulations, à raison de 2 mg/cm$^2$ de peau, sur les avant-bras des personnes témoins (carrés de 6 cm$^2$ environ), et on a mesuré sur ces avant-bras l'écart colorimétrique des valeurs L, a, b (coordonnées trichromatiques mesurées à l'aide d'un colorimètre MINOLTA CM 1000) avant et après application, de manière à déterminer une valeur absolue $\Delta$E qui rend compte de la modification globale de la couleur de la peau après application des formulations :

$$\Delta E = (\Delta L^2 + \Delta a^2 + \Delta b^2)^{1/2}$$

Les résultats sont rassemblés dans le tableau I ci-dessous. Ces résultats montrent clairement que l'évolution moyenne de la couleur de la peau est plus faible dans le cas de l'application de la composition F1 conforme à l'invention, ce qui traduit une meilleure transparence sur la peau.

TABLEAU I

| | FORME DE L'EMULSION | DISPERSION DU PIGMENT | STABILITE | | ΔE | | | |
|---|---|---|---|---|---|---|---|---|
| | | | 45°C | Centrifugation | P1 | P2 | P3 | M[(1)] σ[(2)] |
| F1 | ULTRAFINE, pas de globules huileux visibles au microscope optique (Φ<1 μm) | - fine et homogène<br>- absence d'agglomérats de taille supérieure à 3 μm | stable après 10 jours | stable | 2 | 3,5 | 2,7 | 2,7<br><br>0,7 |
| F2 | globules huileux visibles au microscope optique (Φ>1 μm) | - grossière<br>- nombreux agglomérats de taille supérieure à 10 μm | apparition de deux phases et décantation du pigment après 48 heures | décantation du pigment | 3,3 | 5,7 | 5,3 | 4,8<br><br>1,3 |

[(1)] : moyenne
[(2)] : écart-type

Tous les résultats ci-dessus démontrent que les compositions selon l'invention présentent simultanément les trois avantages que sont la transparence sur la peau, la qualité de dispersion du pigment et la stabilité.

**EXEMPLES 2 à 6**

On donne ci-après d'autres exemples concrets de compositions conformes à l'invention.

| CREME SOLAIRE : | | |
|---|---|---|
| *A* : | - Alcool cétylstéarylique oxyéthyléné (12 OE) (Eumulgin[®] B1 de Sidobre Sinnova) | 3,3 g |
| | - Mono stéarate de glycérol (Tegin[®] 90 de Goldschmidt) | 1,7 g |
| | - Polydécène (Ethyl Flo 362 NF d'Ethyl Corporation) | 10 g |
| | - Dioctylcyclohexane (Cetiol[®] S de Sidobre Sinnova) | 6 g |
| | - Cyclopentadiméthylsiloxane (DC 245 Fluid de Dow corning) | 6 g |
| *B* : | - TiO2 traité stéarate d'aluminium (MT - 100T de Tayca) | 5 g |
| *C* : | - Glycérine | 3 g |
| | - Eau | 15 g |
| *D* : | - Hydroxyéthylcellulose (Natrosol[®] 250HHR d'Aqualon) | 0,6 g |
| | - Eau | qsp 100 g |
| *E* : | - Conservateur | qs |
| *F* : | - Parfum | qs |
| Mode de préparation :<br>1) Préparer le gel *D* sous agitation à 60°C<br>2) Chauffer les phases *A* et *C* à 90°C<br>3) Disperser le pigment *B* dans *A*, puis ajouter la phase *C* sous agitation Moritz (1500 rpm)<br>4) Vers 40°C, rajouter le gel *D*, puis *E* et *F* | | |

| **LAIT SOLAIRE :** | | | |
|---|---|---|---|
| A : | - Alcool cétylstéarylique oxyéthyléné (15 OE) (Mergital® CS 15 de HENKEL) | | 4,4 g |
| | - Mono stéarate de glycérol (Tegin® 90 de Goldschmidt) | | 2,3 g |
| | - Huile de vaseline | | 20 g |
| | - P-méthoxycinnamate de 2-éthyl hexyle (Parsol® MCX de Givaudan) | | 3 g |
| | - Di n-Octyléther (Cetiol® OE de Sidobre Sinnova) | | 7 g |
| B : | -TiO2 traité SiO2/Al2O3 et polydiméthylsiloxane (UVT - M262 de Kemira) | | 2 g |
| C : | - Glycérine | | 5 g |
| | - Eau | | 15 g |
| D : | - Hydroxyéthylcellulose (Natrosol® 250HHR d'Aqualon) | | 0,3 g |
| | - Eau | qsp | 100 g |
| E : | - Conservateur | | qs |
| F : | - Parfum | | qs |

Mode de préparation :
1) Préparer le gel $D$ sous agitation à 60°C
2) Chauffer les phases $A$ et $C$ à 90°C
3) Disperser le pigment $B$ dans $A$,
puis ajouter la phase $C$ sous agitation Moritz (1500 rpm)
4) Vers 40°C, rajouter le gel $D$, puis $E$ et $F$

| **LOTION SOLAIRE :** | | | |
|---|---|---|---|
| A : | - Alcool béhénique oxyéthyléné (1O OE) (Mergital® B10 de Sidobre Sinnova) | | 5 g |
| | - Alcool laurique oxyéthyléné (4 OE) (Brij® 30 d'ICI) | | 5 g |
| | - Polydécène (Ethyl Flo 362 NF d'Ethyl Corporation) | | 10 g |
| | - Huile de vaselline | | 10 g |
| B : | - Glycérine | | 5 g |
| | - Eau | | 15 g |
| C : | - TiO2 traité SiO2/Al2O3 en dispersion aqueuse à 40% (Tioveil AQ de Tioxide) | | 7,5 g |
| | - Acide camphosulfonique (1-4 divinylbenzène) en solution aqueuse à 33% (Mexoryl SX de Chimex) | | 3,03 g |
| | - Triéthanolamine | | 0,6 g |
| | - Eau | qsp | 100 g |
| D : | - Conservateur | | qs |
| E : | - Parfum | | qs |

Mode de préparation :
1) Chauffer les phases $A$ et $C$ à 90°C
2) Ajouter $B$ dans $A$ sous agitation Moritz (1500 rpm)
3) A température ambiante, sous agitation plus faible, rajouter la phase $C$, puis $D$ et $E$.

| CREME SOLAIRE : | | |
|---|---|---|
| A : | - Alcool cétylstéarylique oxyéthyléné (12 OE) (Eumulgin® B1 de Sidobre Sinnova) | 3,6 g |
| | - Mono stéarate de glycérol (Tegin® 90 de Goldschmidt) | 1,4 g |
| | - 4-tertiobutyl 4'méthoxy-dibenzoylméthane (Parsol® 1789 de Givaudan) | 1 g |
| | - Benzoate d'alcools C12/C15 (Finsolv® TN de Finetex)) | 3 g |
| | - 2-cyano 3,3-diphénylacrylate de 2-éthyl hexyle (Uvinul® N539 de BASF) | 2 g |
| | - Huile de vaseline | 15 g |
| B : | - Glycérine | 5 g |
| | - Eau | 15 g |
| C : | - TiO2 traité SiO2/Al203 en dispersion aqueuse à 40% (Tioveil AQ de Tioxide) | 12,5 g |
| D : | - Acide polyacrylique réticulé (Carbopol® 980 de Goodrich) | 0,21 g |
| | - Triéthanolamine | 0,26 g |
| | - Eau | qsp      100 g |
| E : | - Conservateur | qs |
| F : | - Parfum | qs |
| <u>Mode de préparation</u> :<br>1) Préparer le gel D sous agitation à 60°C<br>2) Chauffer les phases A et B à 90°C, ajouter B dans A sous agitaiton Moritz (1500 rpm)<br>3) Disperser le pigment C dans (A+B), puis ajouter le gel D vers 40°C, puis E et F. | | |

| LAIT SOLAIRE : | | |
|---|---|---|
| A : | - Mono stéarate de glycérol oxyéthyléné (20 OE) (Cutina® E24 de Sidobre Sinnova) | 3,3 g |
| | - Mono stéarate de glycérol (Tegin® 90 de Goldschmidt) | 1,7 g |
| | - Huile de vaseline | 15 g |
| | - Adipate de di-isopropyle | 5 g |
| | -2,4,6-tris[p-(2'-éthylhéxyl-1'-oxycarbony)anilino] 1,3,5-triazine (Uvinul®-T150 de BASF) | 1 g |
| B : | - TiO2 traité stéarate d'aluminum (MT - 100T de Tayca) | 3 g |
| C : | - Glycérine | 3 g |
| | - Eau | 15 g |
| D : | - Hydroxyéthylcellulose (Natrosol® 250HHR d'Aqualon) | 0,6 g |
| | - Eau | qsp      100 g |
| E : | - Conservateur | qs |
| F : | - Parfum | qs |
| <u>Mode Opératoire</u> :<br>1) Préparer le gel D sous agitation à 60°C<br>2) Chauffer les phases A et C à 90°C<br>3) Disperser le pigment B dans A,<br>puis ajouter la phase C sous agitation Moritz (1500 rpm)<br>4) Vers 40°C, rajouter le gel D, puis E et F | | |

**Revendications**

1. Composition cosmétique à usage topique, en particulier pour la photoprotection de la peau et/ou des cheveux, comprenant, dans un support cosmétiquement acceptable de type émulsion huile-dans-eau, des nanopigments minéraux à base d'oxydes métalliques à titre d'agents photoprotecteurs, caractérisées par le fait que la taille moyenne des globules qui constituent la phase huileuse de ladite émulsion est comprise entre 100 nm et 1000 nm (nanomètres).

2. Composition selon la revendication 1, caractérisée en ce que ladite taille moyenne est comprise entre 100 et 500 nm.

3. Composition selon l'une quelconque des revendications 1 ou 2, caractérisée en ce qu'au moins 90 % en nombres desdits globules présentent une taille comprise entre 100 et 1000 nm, de préférence entre 100 et 500 nm.

4. Composition selon l'une quelconque des revendications précédentes, caractérisée en ce que la taille moyenne des particules primaires constituant lesdits nanopigments est comprise entre 5 et 100 nm.

5. Composition selon la revendication 4, caractérisée en ce que ladite taille moyenne est comprise entre 10 et 50 nm.

6. Composition selon l'une quelconque des revendications précédentes, caractérisée en ce que lesdits nanopigments sont choisis parmi les oxydes de titane, de zinc, de fer, de zirconium, de cérium et leurs mélanges, enrobés ou non.

7. Composition selon la revendication 6, caractérisée en ce que les nanopigments sont à base d'oxyde de titane, enrobé ou non enrobé.

8. Composition selon la revendication 7, caractérisée en ce que l'oxyde de titane est sous forme rutile, anatase ou amorphe.

9. Composition selon l'une quelconque des revendications précédentes, caractérisée en ce qu'elles contiennent en outre au moins un filtre solaire organique actif dans l'UV-A et/ou l'UV-B.

10. Composition selon l'une quelconque des revendications précédentes, caractérisée en ce qu'elles contiennent en outre au moins un agent émulsionnant non-ionique.

11. Composition selon la revendication 10, caractérisées en ce que la teneur en agent(s) émulsionnant(s) non-ionique est comprise entre 0,5 et 20 % en poids, de préférence entre 2 et 10 % en poids, par rapport au poids total de la composition.

12. Composition selon l'une quelconque des revendications précédentes, caractérisée en ce qu'elles contiennent en outre au moins un adjuvant choisi parmi les épaississants ioniques ou non ioniques, les adoucissants, les antioxydants, les opacifiants, les stabilisants, les émollients, les agents répulsifs contre les insectes, les agents hydratants, les vitamines, les parfums, les conservateurs, les charges, les séquestrants et les colorants.

13. Composition selon l'une quelconque des revendications précédentes, caractérisée en ce que la teneur en poids de la phase aqueuse représente de 50 à 95 %, de préférence de 70 à 90 %, du poids total de la composition.

14. Composition selon l'une quelconque des revendications précédentes, caractérisée en ce que la teneur en poids de la phase huileuse représente de 5 à 50 %, de préférence de 10 à 30 %, du poids total de la composition.

15. Composition selon l'une quelconque des revendications précédentes, caractérisée en ce que la teneur en poids des nanopigments représente de 0,5 à 40 %, de préférence de 1 à 30 %, du poids total de la composition.

16. Procédé de préparation de la composition définie à l'une quelconque des revendications précédentes, caractérisé par le fait qu'il comprend les étapes suivantes :

    (i) on mélange, en présence d'un système émulsionnant non-ionique et sous agitation, une phase grasse d'une part et une phase aqueuse d'autre part, ledit mélange se faisant à une température supérieure à la température d'inversion de phase (TIP) du milieu, de manière à obtenir une émulsion de type eau-dans-huile,

(ii) on ramène la température de l'émulsion ainsi obtenue à une température inférieure à ladite température d'inversion de phase, ce par quoi l'on obtient une émulsion ultrafine de type huile-dans-eau,

(iii) on procède à l'introduction de nanopigments minéraux lors de la mise en oeuvre de l'étape (i) et/ou à l'issue de l'étape (ii).

17. Procédé selon la revendication 16, caractérisé en ce que le ou les agents émulsionnants non ioniques sont choisis, seuls ou en mélanges, parmi les alcools gras polyoxyéthylénés et/ou polyoxypropylénés, et les esters d'acides gras et de polyols, éventuellement polyoxyéthylénés et/ou polyoxypropylénés.

18. Procédé selon la revendication 17, caractérisé en ce que le système émulsionnant présente un HLB global compris entre 9,5 et 11,5 environ, et de préférence proche de 10.

19. Utilisation d'une composition définie à l'une quelconque des revendications 1 à 15 pour la fabrication de formulations photoprotectrices de l'épiderme humain et/ou des cheveux contre le rayonnement ultraviolet, en particulier le rayonnement solaire.

## Claims

1. Cosmetic composition for topical use, especially for photoprotection of the skin and/or hair, comprising, in a cosmetically acceptable vehicle of the oil-in-water emulsion type, inorganic nanopigments based on metal oxides as photoprotective agents, characterized in that the average size of the globules which constitute the oily phase of the said emulsion is between 100 nm and 1000 nm (nanometres).

2. Composition according to Claim 1, characterized in that the said average size is between 100 and 500 nm.

3. Composition according to either of Claims 1 and 2, characterized in that the size of at least 90 %, in numerical terms, of the said globules is between 100 and 1000 nm in size and preferably between 100 and 500 nm in size.

4. Composition according to any one of the preceding claims, characterized in that the average size of the primary particles constituting the said nanopigments is between 5 and 100 nm.

5. Composition according to Claim 4, characterized in that the said average size is between 10 and 50 nm.

6. Composition according to any one of the preceding claims, characterized in that the said nanopigments are chosen from titanium, zinc, iron, zirconium and cerium oxides and mixtures thereof, which are coated or uncoated.

7. Composition according to Claim 6, characterized in that the nanopigments are based on coated or uncoated titanium oxide.

8. Composition according to Claim 7, characterized in that the titanium oxide is in rutile, anatase or amorphous form.

9. Composition according to any one of the preceding claims, characterized in that it also contains at least one organic sunscreen which is active in the UV-A and/or UV-B range.

10. Composition according to any one of the preceding claims, characterized in that it also contains at least one nonionic emulsifying agent.

11. Composition according to Claim 10, characterized in that the content of nonionic emulsifying agent(s) is between 0.5 and 20 % by weight, and preferably between 2 and 10 % by weight, relative to the total weight of the composition.

12. Composition according to any one of the preceding claims, characterized in that it also contains at least one adjuvant chosen from ionic or nonionic thickeners, softeners, antioxidants, opacifiers, stabilizers, emollients, insect repellents, hydrating agents, vitamins, fragrances, preserving agents, fillers, sequestering agents and dyes.

13. Composition according to any one of the preceding claims, characterized in that the weight content of the aqueous phase represents from 50 to 95 %, and preferably from 70 to 90 %, of the total weight of the composition.

14. Composition according to any one of the preceding claims, characterized in that the weight content of the oily phase represents from 5 to 50 %, and preferably from 10 to 30 %, of the total weight of the composition.

15. Composition according to any one of the preceding claims, characterized in that the weight content of the nanopigments represents from 0.5 to 40 %, and preferably from 1 to 30 %, of the total weight of the composition.

16. Process for preparing the composition defined in any one of the preceding claims, characterized in that it comprises the following steps:

> (i) a fatty phase, on the one hand, and an agueous phase, on the other hand, are mixed in the presence of a nonionic emulsifying system and with stirring, the said mixing being done at a temperature above the phase inversion temperature (PIT) of the medium, so as to obtain a water-in-oil type emulsion,
> (ii) the temperature of the emulsion thus obtained is brought down to a value below the said phase inversion temperature, as a result of which an ultrafine oil-in-water type emulsion is obtained,
> (iii) inorganic nanopigments are introduced when step (i) is carried out and/or at the end of step (ii).

17. Process according to Claim 16, characterized in that the nonionic emulsifying agent(s) is (are) chosen, alone or as a mixture, from polyoxyethylenated and/or polyoxypropylenated fatty alcohols and optionally polyoxyethylenated and/or polyoxypropylenated fatty acid esters of polyols.

18. Process according to Claim 17, characterized in that the emulsifying system possesses an overall HLB of between 9.5 and 11.5 approximately, and preferably close to 10.

19. Use of a composition defined in any one of Claims 1 to 15, for the manufacture of formulations for photoprotection of the human epidermis and/or the hair against ultraviolet radiation, in particular solar radiation.

**Patentansprüche**

1. Kosmetische Zusammensetzungen zur topischen Anwendung, insbesondere zum Lichtschutz der Haut und/oder der Haare, die in einem kosmetisch akzeptablen Träger vom Typ einer Öl-in-Wasser-Emulsion anorganische Nanopigmente auf der Basis von Metalloxiden als Lichtschutzmittel enthalten, dadurch gekennzeichnet, daß die mittlere Größe der Kügelchen, die die Ölphase der Emulsion bilden, im Bereich von 100 bis 1000 nm (Nanometer) liegt.

2. Zusammensetzungen nach Anspruch 1, dadurch gekennzeichnet, daß die mittlere Größe im Bereich von 100 bis 500 nm liegt.

3. Zusammensetzungen nach einem der Ansprüche 1 oder 2, dadurch gekennzeichnet, daß mindestens 90 % der Kügelchen eine mittlere Größe im Bereich von 100 bis 1000 nm und vorzugsweise im Bereich von 100 bis 500 nm ausweisen.

4. Zusammensetzungen nach einem der vorhergehenden Ansprüche, dadurch gekennzeichnet, daß die mittlere Größe der Primärteichen, die die Nanopigmente bilden, im Bereich von 5 bis 100 nm liegt.

5. Zusammensetzungen nach Anspruch 4, dadurch gekennzeichnet, daß die mittlere Größe im Bereich von 10 bis 50 nm liegt.

6. Zusammensetzungen nach einem der vorhergehenden Ansprüche, dadurch gekennzeichnet, daß die Nanopigmente unter den Oxiden von Titan, Zink, Eisen, Zirconium, Cer und derer Gemischen ausgewählt sind, wobei sie umhüllt oder nicht umhüllt vorliegen.

7. Zusammensetzungen nach Anspruch 6, dadurch gekennzeichnet, die Nanopigmente auf Titanoxid basieren, wobei sie umhüllt oder nicht umhüllt vorliegen.

8. Zusammensetzungen nach Anspruch 7, dadurch gekennzeichnet, daß das Titanoxid in Form von Rutil, Anatas oder in amorpher Form vorliegt.

9. Zusammensetzungen nach einem der vorhergehenden Ansprüche, dadurch gekennzeichnet, daß sie mindestens ein organisches Sonnenschutzfilter enthalten, das im UV-A- und/oder UV-B-Bereich wirksam ist.

10. Zusammensetzungen nach einem der vorhergehenden Ansprüche, dadurch gekennzeichnet, daß sie ferner mindestens einen nichtionischen Emulgator enthalten.

11. Zusammensetzungen nach Anspruch 10, dadurch gekennzeichnet, daß der Mengenanteil des nichtionischen Emulgators oder der nichtionischen Emulgatoren im Bereich von 0,5 bis 20 Gew.-% und vorzugsweise im Bereich von 2 bis 10 Gew.-%, bezogen auf das Gesamtgewicht der Zusammensetzung, liegt.

12. Zusammensetzungen nach einem der vorhergehenden Ansprüche, dadurch gekennzeichnet, daß sie ferner mindestens einen Hilfsstoff enthalten, der unter den ionischen oder nichtionischen Verdickungsmittel, reizlindernde Mitteln, Antioxidantien, Trübungsmitteln, Stabilisatoren, Emollentien, insektenabwehrende Mitteln, Hydratisierungsmitteln, Vitaminen, Parfums, Konservierungsmitteln, Füllstoffen, Maskierungsmitteln und Färbemitteln ausgewählt ist.

13. Zusammensetzungen nach einem der vorhergehenden Ansprüche, dadurch gekennzeichnet, daß der Gewichtsanteil der wäßrigen Phase im Bereich von 50 bis 95 % und vorzugsweise im Bereich von 70 bis 90 % des Gesamtgewichts der Zusammensetzung liegt.

14. Zusammensetzungen nach einem der vorhergehenden Ansprüche, dadurch gekennzeichnet, daß der Gewichtsanteil der Ölphase im Bereich von 5 bis 50 % und vorzugsweise im Bereich von 10 bis 30 % des Gesamtgewichts der Zusammensetzung liegt.

15. Zusammensetzungen nach einem der vorhergehenden Ansprüche, dadurch gekennzeichnet, daß der Gewichtsanteil der Nanopigmente im Bereich von 0,5 bis 40 % und vorzugsweise im Bereich von 1 bis 30 % des Gesamtgewichts der Zusammensetzung liegt.

16. Verfahren zur Herstellung der Zusammensetzungen nach einem der vorhergehenden Ansprüche, dadurch gekennzeichnet, daß es die folgenden Schritte umfaßt:

   (i) Mischen einer Fettphase und einer wäßrigen Phase in Gegenwart eines nichtionischen Emulgatorsystems unter Rühren zur Bildung einer Emulsion vom Öl-in-Wasser-Typ, wobei das Gemisch bei einer Temperatur über der Temperatur der Phaseninversion ($T_{PI}$) des Mediums gebildet wird;

   (ii) Einstellen der Temperatur der so hergestellten Emulsion auf eine Temperatur unter der Temperatur der Phaseninversion, wodurch eine ultrafeine Emulsion vom Öl-in-Wasser-Typ erhalten wird;

   (iii) Einarbeiten der anorganischen Nanopigmente bei der Durchführung des Schrittes (i) und/oder (ii).

17. Verfahren nach Anspruch 16, dadurch gekennzeichnet, daß der nichtionische Emulgator oder die nichtionischen Emulgatoren unter den polyethoxylierten und/oder polypropoxylierten Fettalkoholen und den Estern von Fettsäuren und Polyolen, die gegebenenfalls polyethoxyliert und/oder polypropoxyliert sind, oder deren Gemischen ausgewählt sind.

18. Verfahren nach Anspruch 17, dadurch gekennzeichnet, daß der HLB-Gesamtwert im Bereich von etwa 9,5 bis 11,5 und vorzugsweise in der Nähe von 10 liegt.

19. Verwendung einer Zusammensetzung nach einem der Ansprüche 1 bis 15 zur Herstellung von Formulierungen zum Lichtschutz der menschlichen Epidermis und/oder der Haare gegen UV-Strahlung, insbesondere gegen Sonnenlicht.